# EUROPÄISCHE PATENTSCHRIFT

(11) **EP 1 185 625 B1**
(45) Veröffentlichungstag und Bekanntmachung des Hinweises auf die Patenterteilung: **17.05.2006**
(21) Anmeldenummer: 00951225.2
(22) Anmeldetag: 19.06.2000
(51) Int. Cl.: C12N 5/06

(54) **VERFAHREN ZUR Herstellung von Neuronalem Zellmaterial**
METHOD FOR PRODUCING Neuronal Cell Material
PROCEDE DE PRODUCTION de MATIERE DE CELLULES NEURONALES

(30) Priorität: 19.06.1999 DE 19928210
(43) Veröffentlichungstag der Anmeldung: 13.03.2002
(73) Patentinhaber: NeuroProgen GmbH Leipzig, 04103 Leipzig (DE)
(72) Erfinder: Peschel, Horst, 51643 Gummersbach (DE)
(74) Vertreter: Keller, Günter
(86) Internationale Anmeldenummer: PCT/DE2000/001932
(87) Internationale Veröffentlichungsnummer: WO 2000/078931

(56) Entgegenhaltungen:
- WO-A-97/02049
- WO-A-99/01159
- WO-A-99/21966
- MAYER-PROSCHEL M ET AL: "ISOLATION OF LINEAGE-RESTRICTED NEURONAL PRECURSORS FROM MULTIPOTENT NEUROEPITHELIAL STEM CELLS" NEURON,US,CAMBRIDGE, MA, Bd. 19, Oktober 1997 (1997-10), Seiten 773-785, XP002914933

## Beschreibung

Die Erfindung betrifft ein Verfahren zur Herstellung von neuronalem Zellmaterial.

Für die Behandlung bestimmter Erkrankungen des Nervensystems wird das Konzept verfolgt, diese Krankheiten durch die Transplantation geeigneter Zellkulturen zu therapieren. Dieser Therapie liegt die Erkenntnis zugrunde, daß bestimmte Krankheiten Funktionsstörungen oder dem Absterben bestimmter Typen von Nervenzellen zuzuordnen sind. So besteht die Möglichkeit, Morbus Parkinson durch Transplantation dopaminerger Neuronen zu therapieren, Morbus Alzheimner durch Transplantation cholinerger Neuronen, Chorea Huntington durch Transplantation striataler Neuronen sowie MSA durch Transplantation striataler und dopaminerger Neuronen.

Problematisch bei diesem Therapieansatz ist zur Zeit, daß die entsprechenden Transplantate nicht in ausreichendem Umfang zur Verfügung stehen, da diese zumeist aus fetalem Gewebe gewonnen werden.

Ein weiteres Problem liegt in möglichen Abstoßungsreaktionen, da verschiedene Zelltypen in dem Transplantat vorhanden sind, die eine Gewebeabstoßung als Immunantwort auslösen können. Daher ist zumeist eine Immunsuppression durch geeignete Arzneimittel notwendig, die jedoch unerwünschte Nebenwirkungen haben können.

Darüber hinaus besteht das Problem, daß die auf bisher bekanntem Wege präparierten Zellen bereits nach wenigen Tagen transplantiert werden müssen, wodurch die Möglichkeiten einer infektiologischen Untersuchung z.B. bezüglich Aids- oder Hepatitis-Viren beschränkt sind.

Der Erfindung liegt die Aufgabe zugrunde, neuronales Zellmaterial zur Verfügung zu stellen, das zur Transplantation geeignet ist oder aus dem Transplantate gewonnen werden können, welches die oben genannten Nachteile nicht aufweist. Des weiteren liegt der Erfindung die Aufgabe zugrunde, ein Verfahren zur Herstellung eines derartigen Zellmaterials bzw. derartiger Transplantate bereitzustellen.

Die Aufgabe wird durch die Merkmale der unabhängigen Ansprüche gelöst. Vorteilhafte Weiterbildungen sind den Unteransprüchen zu entnehmen.

Die Erfindung basiert auf dem Konzept, Progenitorzellen in Kultur zu halten und zu vermehren. Diese Vermehrung kann durch die Einwirkung geeigneter Wirkstoffe, insbesondere Eiweiße, derart gesteuert werden, daß diese Progenitorzellen nach Transplantation oder allgemein nach Kontaktierung mit einem geeignetem Substrat wie beispielsweise Zellmaterial oder einem Trägermaterial nur noch oder überwiegend in einen bestimmten Zelltyp (dopaminerge Neuronen) differenzieren. Durch diese Behandlung können Zellkulturen erhalten werden, die praktisch ausschließlich aus den direkten Vorläufern der gewünschten neuronalen Zellen bestehen und insbesondere immunkompetente gliale Zellen nur noch in Anteilen enthalten, die keine physiologische Wirkung mehr haben, insbesondere nicht mehr nachweisbar sind. Hierdurch können geeignete und bezüglich der vorhandenen Zelltypen besser definierte Transplantate in praktisch unbegrenztem Ausmaß hergestellt werden.

So kann Zellmaterial erhalten werden, das praktisch ausschließlich dopaminerge Neuronen enthält, d.h. der Anteil der genannten Neuronen an dem Zellmaterial beträgt größer 90%, vorzugsweise größer 95% bzw. enthält keine physiologisch wirksamen Anteile anderer Zellen, insbesondere glialer Zellen.

Die Progenitorzellen, durch deren Vermehrung und Selektion Zellkulturen bzw. transplantationsfähiges Zellmaterial gewonnen wird, können sowohl aus fetalem als auch aus adultem neuronalem Zellmaterial (Gehirn oder Rückenmark) gewonnen werden. Das adulte Zellmaterial wird bisher vorteilhafterweise aus periventrikulären oder hippokampalen Abschnitten präpariert.

Der Entnahmeort der fetalen Zellen kann sich nach dem Zielgewebe richten, d.h. Progenitorzellen werden aus den Arealen entnommen, in denen die gewünschten Neuronen später vorkommen, z.B. dopaminerge Neuronen aus Mittelhirngewebe. Es können bei geeigneter partieller Differenzierung mittels des erfindungsgemäßen Verfahrens jedoch insbesondere auch verschiedene Progenitorzellen aus Gewebe ein- und derselben Entnahmestelle gewonnen werden, z.B. auch aus Stammzellen des Blutes aus Nabelschnurgewebe.

Die Isolierung und Kultivierung von neuronalen Progenitorzellen aus dem Nagetierhirn ist mehrfach beschrieben worden (Daadi und Weiss, J. Neurosci 1999; Magrassi et al., Development 1998; 54:107-115; Ptak et al., Cell Transplant 1995; 4:299-310; Liepelt et al., Brain Res Dev Brain Res 1990; 51:267-278). Die Autoren konnten Progenitorzellen aus verschiedenen Arealen im Gehirn präparieren. Des weiteren wurden humane Progenitorzellen aus fetalem kortikalem Gewebe (bzw. dem gesamten Gehirn) präpariert (Buc-Caron, Neurobiol Dis 1995; 2:37-47; Svandsen CN et al., Exp Neurol 1997; 148:135-146; Sah et al., Nat Biotechnol 1997; 15:574-580; Chalmers-Redman et al., Neuroscience 1997; 76:1121-1128). Bezüglich der Art und Weise der Präparation der Progenitorzellen wird auf die oben genannten Druckschriften hiermit vollinhaltlich Bezug genommen.

Die Bereitstellung von transplantationsfähigem neuronalem Zellmaterial erfolgt gemäß der Erfindung durch ein Verfahren, das eine Expansion der mittelbar oder unmittelbar gewonnenen humanen Progenitorzellen, eine partielle in vitro Differenzierung und eine Selektionierung umfaßt, wobei die letztlich erhaltenen neuronalen Kulturen insbesondere gegebenenfalls ohne Zugabe weiterer Faktoren oder dauerhafter genetischer Manipulationen mit hohem Prozentsatz in den gewünschten Zelltyp ausdifferenzieren können.

Gegebenenfalls kann nach der partiellen Differenzierung oder der Selektionierung der Progenitorzellen eine erneute Expansion des Zellmaterials erfolgen.

Die partielle Differenzierung und die Selektionierung können mehrmals wiederholt werden, wobei die Art und Weise der Durchführung sich jeweils unterscheiden kann.

Durch die Erfindung ist es insgesamt möglich semiunreife neuronale Progenitorzellen soweit zu selektionieren und differenzieren, daß nach Zugabe von Nährmedien bzw. nach Transplantation ausschliesslich oder überwiegend ein spezifischer Zelltyp ausdifferenziert.

Die Expansion kann insbesondere unter gleichzeitiger Anwendung der Prozesse der Hypoxie, Priming, gegebenenfalls unter vermindertem Sauerstoffgehalt der Atmosphäre, transienten oder nichttransienten genetischen Manipulation und/oder Behandlung mit exogenen Faktoren, insbesondere unter vermindertem Sauerstoffgehalt der Atmosphäre, erfolgen. Die Verfahrensschritte werden später detailierter beschrieben.

Die Selektionierung erfolgt durch Subklonierung unter Hypoxie.

Das Verfahren kann insbesondere so ausgeführt werden, daß nach der Expansion der frisch gewonnen Progenitorzellen eine selektive Expansion des gewünschten Zelltyps vorgenommen wird. Die selektive Expansion kann durch Veränderung des Sauerstoffgehaltes, die Applikation geeigneter Mitogene oder eine partielle Differenzierung (Priming) mittels exogener Faktoren, wahlweise jeweils unter vermindertem Sauerstoffgehalt der Atmosphäre, erfolgen. Die Selektionierung der "determinierten" Progenitoren erfolgt durch Subklonierung unter vermindertem Sauerstoffgehalt der Atmosphäre. Der Subklonierung folgt eine erneute Expansion und ggf. eine erneute partielle Differenzierung durch eine der oben angegebenen Methoden (Hypoxie, Priming, bzw. Behandlung mit exogenen Faktoren), insbesondere durch Behandlung mit exogenen Faktoren oder durch Priming, wahlweise jeweils unter vermindertem Sauerstoffgehalt der Atmosphäre. Die erste oder zweite partielle Differenzierung kann auch durch eine transiente oder nichttransiente genetische Manipulation erfolgen bzw. unterstützt werden. Ergänzend zu einer Verminderung des Sauerstoffgehaltes können die Verfahrensschritte auch unter Bedingungen durchgeführt werden, die Veränderungen simulieren, die durch diesen veränderten Sauerstoffgehalt induziert werden (z. B. mit Hemmern der mitochondrialen Atmung wie Rotenon, MPP+ oder Malonat).

Die Expansion nach den einzelnen Behandlungen sollte vorzugsweise jeweils von einer einzelnen Zelle ausgehen.

Der Erfolg der Selektionierung dieser determinierten Progenitorzellen d. h. die Charakterisierung der erhaltenen Neuronenpopulationen erfolgt nach kompletter Differenzierung (in vitro oder in vivo), vorteilhafterweise mit zytometrischen, biochemischen, molekularbiologischen, immunhistochemischen und elektrophysiologschen Methoden. Diese sind für die verschiedenen Neuronenpopulationen der einschlägigen Fachliteratur zu entnehmen.

### Expansion unter hypoxischen Bedingungen:

Es hat sich herausgestellt, daß eine erhebliche Verbesserung der Teilungsrate durch eine Absenkung des Luftsauerstoffgehalts und/oder eine Erhöhung des Stickstoffgehaltes erzielt werden kann, wobei hierdurch zugleich die Anteile spezieller neuronaler Progenitorzellen, insbesondere der dopaminergen Neuronen, erhöht werden, also eine Differenzierung erfolgt. Beispielhaft sei hier eine Erniedrigung der Sauerstoffspannung von 21 % (Raumluft) auf 10%, vorzugsweise 5%, besonders bevorzugt 1 % genannt, gegebenenfalls in Verbindung mit einer gleichzeitigen Erhöhung des Stickstoffgehaltes, wobei gegebenenfalls auch andere Gase zusätzlich eingesetzt werden können, z.B. 1-10, vorzugsweise ca. 5 % CO₂. Die Absenkung des Luftsauerstoffgehalts erfolgt unter Bedinungen, unter denen sich das Zellmaterial vermehrt. Durch diese Technik der Kultivierung können mammale, insbesondere humane Progenitorzellinien auch aus den verschiedenen Hirnarealen (auch Mittelhirngewebe) etabliert werden.

Bei der Expansion eingesetzte exogene Faktoren (siehe unten) können einzeln oder in Kombination jeweils in Konzentrationen von 4000 bis 0,01 ng/ml, vorzugsweise 500 bis 1 ng/ml, besonders bevorzugt 100 bis 2 ng/ml der Expansionslösung vorliegen, ohne hierauf beschränkt zu sein.

### Partielle Differenzierung durch Priming:

Priming umfaßt, daß die (monoklonalen) neuronalen Progenitorzellen mit LIF, GDNF und /oder einem oder mehreren der Interleukine IL 1-11 behandelt werden, der/die die Differenzierung in die gewünschte Nervenzellpopulation begünstigt. Zu diesem Zweck können auch sogenannte konditionierte Medien eingesetzt werden, d.h. Kulturmedien, die zur Kultivierung insbesondere der Neuronen des Zielgebietes der gewünschten Nervenzellenpopulation (z.B. Striatum bei dopaminergen Neuronen) oder Glialzellen verwendet werden oder aus primären Kulturen dieser Zellen erhalten werden. Diese Medien enthalten die Eiweisse, die von den jeweiligen Zellen sezerniert wurden. Diese exogenen Faktoren werden dann zu einem Zeitpunkt (vorzugsweise nach einigen Stunden) wieder entzogen, zu welchem die Zellen wieder in einen Zustand entdifferenzieren, in dem die weitere Expansion möglich ist. Derart rückumgewandelte Progenitorzellen werden als geprimte Zellen bezeichnet. Bei einer zweiten Exposition mit wirksamen endogenen Faktoren (Z.B. nach Transplantation in ein adultes Gehirn) kommt es dann zu einer vielfach schnelleren Differenzierung. Bei (erneuter) Expansion werden dann geprimte (ggf. auch monoklonale) Zellinien gewonnen, die bereits Gene exprimieren, die eine höhere Spezifität gewährleisten. Dieser Schritt kann beliebig oft wiederholt werden, wobei die Zellen jeweils anderen Faktoren oder den gleichen Faktoren in anderen Konzentrationen ausgesetzt werden können.

Für das Priming wird LIF, GDNF und /oder eines oder mehrere der Interleukine IL 1-11 verwendet.

Daneben können jeweils Kombinationen, insbesondere der unten genannten, Zytokine und Wachstumsfaktoren, Zytokine und Transkriptionsfaktoren, Zytokine und Neurotransmittern, Zytokine und Hormone, Zytokine und Gangliosiden, Zytokine und konditionierte Medien, Wachstumsfaktoren und Transkriptionsfaktoren, Wachstumsfaktoren und Neurotransmitter, Wachstumsfaktoren und Hormone, Wachstumsfaktoren und Gangliosiden, Wachstumsfaktoren und konditionierte Medien, Transkriptionsfaktoren und Neurotransmittern, Transkriptionsfaktoren und Hormone, Transkriptionsfaktoren und Ganglioside, Transkriptionsfaktoren und konditionierte Medien, Neurotransmitter und Hormone, Neurotransmitter und Gangliosiden, Neurotransmitter und konditionierte Medien verwendet werden.

Insbesondere können auch Kombinationen von Zytokinen und Wachstumsfaktoren zusammen mit Transkriptionsfaktoren oder Neurotransmittern oder Hormonen oder Ganglioside oder konditionierten Medien eingesetzt werden. Es können auch Kombinationen von Zytokinen und Transkriptionsfaktoren zusammen mit Neurotransmittern oder Hormonen oder Ganglioside oder konditionierten Medien eingesetzt werden. Es können auch Kombinationen von Zytokinen und Neurotransmitter zusammen mit Hormonen oder Gangliosiden oder konditionierten Medien eingesetzt werden. Es können auch Kombinationen von Wachstumsfaktoren und Transkriptionsfaktoren zusammen mit Neurotransmittern oder Hormonen oder Gangliosiden oder konditionierten Medien eingesetzt werden. Es können auch Kombinationen von Wachstumsfaktoren und Neurotransmittern zusammen mit Hormonen oder Ganglioside oder konditionierten Medien eingesetzt werden. Es können auch Kombinationen von Wachstumsfaktoren und Hormonen zusammen mit Ganglioside oder konditionierten Medien eingesetzt werden. Es können auch Kombinationen von Wachstumsfaktoren und Gangliosiden zusammen mit konditionierten Medien eingesetzt werden.

Als Wachstumsfaktoren werden einer oder mehrere aus der Gruppe epidermal growth factor (EGF), insbesondere EGF1, EGF2, EGF3 mit den Subgruppen α und β, transforming growth factor (TGF) α und β, LIN-3-Protein, fibroblast growth factor (FGF), FGF1 und FGF2, nerve growth factor (NGF), brain-derived neurotrophic factor (BDNF), Neurotrophine (NT), insbesondere NT-3, NT-4, NT-5, NT-6, insulin-like growth factors (IGF), insbesondere IGF-1 und IGF-2, glial cell line-derived neurotrophic factor (GDNF), Neurturin (NTN), Persephin (PSP), vascular endothelial growth factor (VEGF), einschließlich deren Untergruppen oder Faktoren ähnlicher Wirkung verwendet.

Als Zytokine werden einer oder mehrere aus der Gruppe Interleukine (IL 1-16), leukemia inhibitory factor (LIF), ciliary neurotrophic factor (CNTF), tumor necrosis faktor (TNF), insbesondere TNF-α, Interferone (IFN), insbesondere IFN-α, makrophage inhibitory oder stimulating factors, insbesondere macrophage migration inhibitory factor (MIF), mitochondrial import stimulation factor (MSF) und Retinsäure eingesetzt.

Als Neurotransmitter werden einer oder mehrere aus der Gruppe Dopamin, Acetylcholin, GABA, Glutamat, Glycin, Taurin, Prolin, Noradrenalin, Serotonin und Neuropeptide, insbesondere Substanz P und Enkephalin, eingesetzt.

Die Neurotransmitter werden allein oder in Gegenwart von Wachstumsfaktoren und/oder Zytokinen eingesetzt werden.

Einzeln oder in Kombination mit den oben genannten Stoffen oder Stoffkombinationen können Hormone wie Wachstumshormone, Schilddrüsenhormone (insbesondere zur Differenzierung der Progenitorzellen zu dopaminergen Neuronen), Steroidhormone oder Ganglioside, jeweils einschließlich deren Derivate, eingesetzt werden.

Zur Erzeugung von dopaminergen Neuronen werden GDNF, LIF und eine oder mehrere von IL1-11 einzeln oder in Kombination eingesetzt, insbesondere die Kombination IL-1, GDNF, LIF, IL-11, jeweils einschließlich deren Untergruppen.

Die exogenen Faktoren können einzeln oder in Kombination jeweils in Konzentrationen von 25,000 bis 0,005 ng/ml, vorzugsweise 1000 bis 0,1 ng/ml, besonders bevorzugt 100 bis 1 ng/ml Expansionslösung eingesetzt werden, ohne hierauf beschränkt zu sein.

Insbesondere können zur Differenzierng jeweils IL-1 in Konzentrationen von 0,005 bis 10 ng/ml, vorzugsweise 0,01 bis 2 ng/ml, besonders bevorzugt 0,05 bis 0,25 ng/ml eingesetzt werden. IL-11 und LIF können in Konzentrationen von 0,01 bis 100 ng/ml, vorzugsweise 0,1 bis 20 ng/ml, besonders bevorzugt 0,5 bis 2,5 ng/ml eingesetzt werden. GDNF kann in Konzentrationen von 1 bis 25.000 ng/ml, vorzugsweise 1-10 bis 5000 ng/ml, besonders bevorzugt 1-100 bis 2.500 ng/ml eingesetzt werden.

Die Faktoren können auch in Kombination in diesen Konzentrationen eingesetzt werden. Die einzusetzenden Konzentrationen sind jedoch nicht auf die oben genannten Werte beschränkt und können unter anderem können in Abhängigkeit von den jeweils anderen eingesetzten Faktoren variieren.

### Partielle Differenzierung durch Transfektion:

Insbesondere im Rahmen des oben beschriebenen Primings aber auch unabhängig hiervon können auch genetische Manipulationen eingesetzt werden. Durch die Transfektion der Progenitorzellen mit Genen, die in der Entwicklung der spezifischen Neuronen relevant sind, wird eine Differenzierung in den gewünschten Zelltyp begünstigt. Auch eine vorübergehende Expression dieser Gene, welche das Erbmaterial der Zellen nicht verändert und keine Fremdgene nach der Transplantation in das Wirtsgehirn einschleust, determiniert das weitere Schicksal.

Die Entwicklung von dopaminergen Neuronen kann insbesondere durch Transfektion von Genen gesteuert werden, die Mitglieder der Steroid- und Thyroid-Hormon-Rezeptor-Familie wie Tyrosinhydroxylase, Nurr-1 und/oder Nurr-77 Rezeptoren kodieren. Insbesondere können auch Gene des vesikulären Monoamintransporters oder des Dopamintransporters eingesetzt werden. Generell können Gene verwendet werden, die für dopaminerge Neuronen spezifisch sind.

Die entsprechenden cDNA's mit den oben genannten Genen, sind aus der Literatur bekannt und verfügbar. Die Transfektion erfolgt mit den in der Literatur angegebenen Standardverfahren. So ist eine transiente oder stabile Transfektion der Progenitorzellen möglich.

### Selektionierung durch Subklonierung:

Für die Selektionierung ist insbesondere die Subklonierung geeignet. Mittels Subklonierung lassen sich aus heterogenen Zellkulturen (in diesem Fall die gewonnenen neuronalen Progenitorzellen) Zellinien generieren, die aus einer einzelnen dieser Progenitorzellen entstanden sind (monoklonale oder gegebenenfalls polyklonale Zellinien). Somit kann ohne Zugabe exogener Faktoren die Heterogenität dieser Zellen minimiert werden.

Die Subklonierung der Progenitorzellen kann durch Endverdünnung der Zellsuspension, durch Mikromanipulation oder Fluoreszenzaktivierte Zellsortierung ("fluorescence-activated cell sorting"; FACS) nach Markierung der vitalen Zellen oder durch magnetische Aufkonzentrierung nach Markierung der Zellen mit supraparamagnetischen Partikeln erfolgen.

Die Subklonierung der Progenitorzellen durch Endverdünnung der Zellsuspension erfolgt beipielsweise, indem die Zellsuspension soweit verdünnt wird, daß lediglich eine Zelle in jedem Kulturgefäß verbleibt. Die so ausplatierten Zellen werden expandiert, wodurch man monoklonale Zellinien erhält. Dabei werden die Medien vorzugsweise mit mitogenen Substanzen versetzt (siehe oben angegebene Wachstumsfaktoren), um eine Vermehrung aus einer Einzelzelle zu erreichen. Die Expansion, Differenzierung und Charakterisierung erfolgt dann im weiteren wie bereits oben für polyklonale Progenitorzellsuspensionen beschrieben.

Die Subklonierung der Progenitorzellen durch Mikromanipulation nach Fluoreszenz-Markierung der vitalen Zellen kann erfolgen, indem die lebenden Zellen mit einem für die jeweilige Zellpopulation spezifischen Marker gefärbt werden. Die markierten Zellen werden dann unter einem Fluoreszenz-Mikroskop mittels Mikrowerkzeugen (z.B. Glaskapillaren) verlesen, so daß nur markierte Zellen eines Typs im Kulturgefäß verbleiben. Die so ausplatierten Zellen werden expandiert, wodurch man Zellinien erhält, die lediglich einen Zelltyp enthalten.

Zur Expansion werden die Medien vorzugsweise mit mitogenen Substanzen versetzt (siehe oben angegebene Wachstumsfaktoren), um eine Vermehrung aus einer Einzelzelle zu erreichen. Die Expansion, Differenzierung und Charakterisierung erfolgt dann im weiteren wie bereits oben für die polyklonale Progenitorzellsuspension beschrieben. Eine Subklonierung mittels Endverdünnung (s.o.) kann ggf. angeschlossen werden. Als Marker für dopaminerge Zellen werden die Zellen beispielsweise mit dem Gen für das grün fluoreszierende "enhanced green-fluorescence protein" (EGFP), welche unter der Kontrolle spezifisch dopaminerger Promotoren exprimiert werden (Tyrosinhydroxylase- und Dopamintransporter-Promotor), vorübergehend transfiziert. Die grün leuchtenden Zellen exprimieren entsprechend die beiden genannten spezifischen dopaminergen Proteine und werden anschließend wie beschrieben kloniert werden. Weiterhin kann die Markierung der lebenden Zellen mittels fluoreszierender Antikörper erfolgen. Für dopaminerge Zellen können Antikörper gegen den Dopamintransporter verwendet werden.

Die Subklonierung der Progenitorzellen durch Fluoreszenzaktivierte Zellsortierung kann mittels Durchflußzytometrie ("fluorescence-activated cell sorting"; FACS) nach Fluoreszenz-Markierung der vitalen Zellen erfolgt, indem die lebenden Zellen mit einem für die jeweilige Zellpopulation spezifischen Marker gefärbt werden. Die markierten Zellen werden dann unter mittels FACS-Gerät nach den in der Literaut angegebenen Standardverfahren sortiert(zur Übersicht: Orfao und Ruiz-Arguelles, Clin Biochem 1996;29:5-9), so daß nur markierte Zellen eines Typs im Kulturgefäß verbleiben. Die so ausplatierten Zellen werden expandiert, wodurch man Zellinien erhält, die lediglich einen Zelltyp enthalten. Zur Expansion werden die Medien vorzugsweise mit mitogenen Substanzen versetzt (siehe oben angegebene Wachstumsfaktoren), um eine Vermehrung aus einer Einzelzelle zu erreichen. Die Expansion, Differenzierung und Charakterisierung erfolgt dann im weiteren wie bereits oben für die polyklonale Progenitorzellsuspension beschrieben. Eine Subklonierung mittels Endverdünnung (s.o.) kann ggf. angeschlossen werden. Als Marker für dopaminerge Zellen werden die Zellen beispielsweise mit dem EGFP Gen welches unter der Kontrolle spezifisch dopaminerger Promotoren exprimiert wird (Tyrosinhydroxylase- und Dopamintransporter-Promotor), vorübergehend transfiziert. Die grün leuchtenden Zellen exprimieren entsprechend die beiden genannten spezifischen dopaminergen Proteine und werden anschließend wie beschrieben kloniert werden. Weiterhin kann die Markierung der lebenden Zellen mittels fluoreszierender Antikörper erfolgen. Für dopaminerge Zellen können beispielsweise Antikörper gegen den Dopamintransporter verwendet werden.

Die Subklonierung der Progenitorzellen durch magnetische Aufkonzentrierung nach Markierung der Zellen mit supraparamagnetischen Partikeln erfolgt, indem die lebenden Zellen mit einem für die jeweilige Zellpopulation spezifischen Marker magnetisch markiert werden. Es stehen verschiedene Partikel zur Verfügung (Basic microbeads - dextran beschichtet mit freien Aminen, 50nm, Miltenyi Biotech; Amino/Carboxy beads, 110-140 nm, Immunicon Corp.; Streptavidin/Biotin beschichtet, Miltenyi Biotech oder Immunicon Corp.). Diese Partikel können mit Liganden für spezifische Oberflächenproteine (z. B. Dopamin-D2-Rezeptoren, Dopamintransporter oder andere Transporter) beladen werden. Anschliessend können Zellen, die an diese Partikel binden, mittels magnetischer Säulen isoliert werden.

Die ausplatierten Zellen werden expandiert, wodurch man Zellinien erhält, die lediglich einen Zelltyp enthalten. Zur Expansion werden die Medien vorzugsweise mit mitogenen Substanzen versetzt (siehe oben angegebene Wachstumsfaktoren), um eine Vermehrung aus einer Einzelzelle zu erreichen. Die Expansion, Differenzierung und Charakterisierung erfolgt dann im weiteren wie bereits oben für die polyklonale Progenitorzellsuspension beschrieben. Eine Subklonierung mittels Endverdünnung (s.o.) kann ggf. angeschlossen werden. Die Markierung der lebenden Zellen erfolgt mittels Antikörper, die mit magnetischen "Mikrobeads" gekoppelt wurden. Für dopaminerge Zellen können Antikörper gegen den Dopamintransporter verwendet werden. Weiterhin ist die Verwendung von niedermolekularen Liganden zelltyp-spezifischer Proteine möglich. Für dopaminerge Neurone würden sich Liganden für den Dopamin-D2-Rezeptor (Benzamid- oder Spiperonderivate) oder den Dopamintransporter anbieten (Kokainderivate).

Bei allen diesen Verfahren werden die Zellen in einem Stadium subkloniert, in dem eine möglichst weitgehende Differenzierung erfolgt ist, ohne daß die Teilungsfähigkeit der Zellen gemindert wird, also nach einem Priming, genetischer Manipulation, Veränderung der Atmosphäre oder Behandlung mit exogenen Faktoren.

Die oben beschrieben Schritte der partiellen Differenzierung, Selektionierung (Klonierung) und Expansion können bei Bedarf kombiniert und wiederholt angewandt werden.

### Allgemeine Verfahrensdurchführung

Nach der Präparation des Hirngewebes erfolgt die Homogenisierung. Vorteilhafterweise wird das Gewebe mit einem proteolytisch wirkendem Enzym, insbesondere einer Serin-Protease wie Trypsin, vorzugsweise in einer Konzentration von 50 bis 500 mg/ml versetzt, um den Gewebeverband zu lockern.

Anschließend wird das so präparierte Gewebe vorteilhafterweise mit einer DNase-Lösung versetzt, die vorzugsweise eine Konzentration von 0,5 bis 20 mg, vorzugsweise 2 bis 6 mg DNase auf 100 ml Lösung enthält. Vorzugsweise wird DNase I eingesetzt. Die DNase-Lösung wird für 2 bis 30 Minuten, vorzugsweise ca. 10 Minuten inkubiert, um extrazelluläre DNA zu verdauen, da diese das Zellüberleben und die Homogenisierung beeinträchtigen kann.
Anschließend werden die angedauten Gewebeteile durch mehrmaliges Aufziehen in eine Glas-Pasteupipette homogenisiert.

Anschließend wird das Gewebe mit einer wirksamen Menge eines Expansionsmediums zur Vermehrung der Progenitorzellen versetzt und in Kulturflaschen expandiert, wie dies aus der angegebenen Literatur bekannt ist.

Das Expansionsmedium kann neben den expansionsfördernden (mitogenen) Faktoren 10 bis 60 %, vorzugsweise 30 bis 45% F-12 Medium, 30 bis 75 %, vorzugsweise 45 bis 70% Dulbecco's Modified Eagle's Medium (DMEM; glucosefrei oder mit geringen oder hohen Glucosegehalt), wirksame Mengen eines geeigneten Antibiotikums, wie z.B. Penicillin oder Streptomycin, in einer Konzentration von 50 bis 250 I. E/ml für Penicillin und 50 bis 250 µg/ml für Streptomycin, vorzugsweise 150 I. E./ml für Penicillin und 150 µg/ml für Streptomycin enthalten.

Die Expansionsmedien können des weiteren ein oder mehrere Substanzen aus der Gruppe Transferrin, biogene Amine, insbesondere Diamine wie z.B. 1,4-Diaminbutan (Putrescin), bakterizidwirkende Reduktionsmittel wie Natriumselenit, Gestagene, insbesondere Progestativa wie Progesteron oder progesteronartige Wirkstoffe, sowie Insulin enthalten. Alternativ können verschiedene Supplemente, die aus Serum extrahiert werden, wie z.B. das kommerziell erhältliche B-27 Supplement (Firma Gibco), verwandt werden.

Die Expansion wird in veränderter Atmosphäre mit reduziertem Luftsauerstoffgehalt durchgeführt. Hierzu werden die Zellen in einer entsprechenden Kammer gelagert, in der eine exakte Sauerstoffkonzentration gewährleistet ist. Je nach gewünschtem Zelltyp wird der Sauerstoffgehalt zwischen 1 % und 10 % variiert.

Für die hier zu klonierenden Zellkulturen können insbesondere mitogene Faktoren dem Medium zugesetzt werden. Dazu werden vorteilhafterweise oben genannte Faktoren verwandt. Auch die Subklonierung erfolgt vorzugsweise unter geänderter Atmosphäre. Es hat sich als vorteilhaft erwiesen, die Sauerstoffkonzentration sehr individuell auf die isolierten Zellen einzustellen, insbesondere auf Werte zwischen 1 % und 15 %.

Nach ausreichender Expansion der Zellklone erfolgt die Selektionierung und partielle Differenzierung der Zellen zu transplantationfähigen Zellkulturen.

Die Transfektion der Zellen mit Genen kann unter der Zugabe geeigneter Mengen, beispielsweise von 0,5 µg von plasmider DNA je ml Inhalt eines Kulturgefässes und 3 µl je µg DNA von TransFast (Pro mega)-Lösung auf 1 ml des Expansionsmediums gemäß den Bestimmungen des Lieferanten erfolgen. Diese Lösung kann bei 37°C inkubiert und anschließend der Gewebeprobe zugegeben werden. Zur Identifizierung der differenzierten Neuronen können DNA und Lipofektin inkubiert werden und anschließend im Medium I differenziert werden. Die Zellen können dann auf übliche Weise geerntet und weiterbehandelt werden.

Die Messung der Proliferation durch die Inkorporierung von [³H] Thymidin und die Proteinbestimmung sowie die Messung der Proliferation und Vitalität durch Flusszytometrie erfolgen nach üblichen Verfahren.

Zur Differenzierung der Progenitorzellen können die Zellen in Vitro durch Platierung auf Poly-L-Lysin-beschichtete Abdeckstreifen oder 48-Lochplatten in neurobasalem Medium (Gibco) aufgebracht werden. Die Medien können mit FCS, Zytokinen und/oder striatal-konditionierten Medien versetzt werden. Es können beispielsweise die Zytokine IL-1B, IL-11, LIF, GDNF oder andere exogene Faktoren wie unter dem Abschnitt "Priming" beschrieben, verwendet werden. Die Zellen werden für 7 bis 10 Tage bei 37°C in einer befeuchteten Atmosphäre vor der Fixierung und weiteren Untersuchungen differenziert.

Die funktionelle Integrität der Neuronen, z.B. DA und GABA-Neuronen, kann durch Messung der Aufnahme von tritiierten Neurotransmittern erfolgen. Nach der Präinkubation für 10 Minuten in einem Inkubationspuffer enthaltend 100 µM Pargylin, 1mM Ascorbat und 2mM β-Alanin (und zur Bestimmung der unspezifischen Aufnahme: 3µM GBR12909 und 1mM 2,4-Diamino-n-buttersäure; DABA) können 50 nM [³H]DA, [³H]Cholin oder [³H]GABA für 15 Minuten bei 37°C zugegeben werden. Die Aufnahme kann durch Waschen der Platten mit kaltem PBS gestoppt werden und die verbleibende Radioaktivität des Zell-Lysates kann durch Messung unter Verwendung flüssiger Szintillationszählung erfolgen. Die spezifische Aufnahme kann als die Differenz zwischen der Aufnahme in Abwesenheit (gesamt) und der bei Anwesenheit von GBR12909 und DABA (unspezifisch) erfolgten Aufnahme bestimmt werden.

Die Erfindung sei nachfolgend beispielhaft beschrieben.

### Ausführungsbeispiel

Zur Gewinnung von Progenitorzellen zur Herstellung humaner neuronaler Zellen eines bestimmten Typs bzw. eines entsprechenden transplantationfähigen neuronalen Zellmaterials wird das Gehirn eines humanen Fötus verwendet, dessen Hirnhäute entfernt wurden. Die Präparation der Gehirnsubstanz erfolgt unter Kühlung, beispielsweise in einer Petriglasschale auf Eis. Die Hirnanteile, aus denen die Progenitorzellen gewonnen werden sollen, werden mit zwei Skalpellen präpariert, wobei die periventrikulären Abschnitte mit präpariert werden. Die präparierten Gehirnteile werden in sterilen Plastikröhrchen mit 12 bis 14 ml einer Dissektionslösung auf Eis überführt.

Die Dissektionslösung enthält 98 % calcium- und magnesiumfreies Hank's buffered salt solution (HBSS), 150 I. E./ml Penicillin/150 µg/ml Streptomycin sowie 4500 mg/l D-Glucose.

Zur Expansion der neuronalen Progenitorzellen wird die das neuronale Zellmaterial enthaltende Dissektionslösung zentrifugiert und der Überstand abgesaugt. Das separierte Gewebe mit 1 ml Trypsinlösung (250 mg Trypsin in 100 ml HBSS, steril filtriert und zur Lagerung in 1 ml-Proben bei - 20° C eingefroren) versetzt und aufgeschüttelt. Die Lösung wird für 30 Minuten bei Raumtemperatur inkubiert. Die Probe wird anschließend zentrifugiert und der Überstand von dem Gewebe abgesaugt.

Anschließend wird das so erhaltene Pellet DNAse-Lösung (4 mg DNase I, 15 mg Sojabohnen Trypsin Inhibitor, 188.2 mg MgSO₄ auf 100 ml HBSS, steril filtriert und zur Lagerung in 2 ml-Proben bei -20° C eingefroren) versetzt und aufgeschüttelt.

Die Lösung wird für zehn Minuten bei 37° C inkubiert, anschließend zentrifugiert und der Überstand von dem Gewebe abgesaugt.

Das Gewebe wird anschließend mit 3 bis 5 ml Expansionsmedium I oder Expansionsmedium II zur Zellvermehrung versetzt.

Das Expansionsmedium I besteht aus 32 % F-12 Medium, 65 % Dulbecco's Modified Eagle's Medium (DMEM) (4500 mg/l D-Glukose), 2 % B-27 Ergänzungslösung (Firma Gibco), 150 I. E./ml Penicillin/150 µg/ml Streptomycin sowie den Wachstumsfaktoren EGF (20 ng/ml), wahlweise auch EGF (20 ng/ml) und FGF 2 (20 ng/ml).

Das Expansionsmedium II enthält 45.1 % F-12 Medium, 45.1 % DMEM, 1 ml Transferrin-Stammlösung (100 mg Transferrin auf 10 ml HBSS), 1 ml Putrescine-Stammlösung (bestehend aus einer 126 mg Putrescin-Hydroclorid auf 100 ml HBSS, entsprechend einer Putrescin-Konzentration von 60 µM), 1 ml Penicillin/Streptomycin, 200 µl Na-Selenit-Stammlösung (1 mg Natrium-Selenit, 11.6 ml H₂O 0,3 ml dieser Lösung auf 9,7 ml HBSS ergibt die Stammlösung), 200 µl Progesterone-Stammlösung (1 mg Progesterone auf 3,2 ml Ethanol, 50 µl der ethanolischen Lösung auf 4,95 ml HBSS ergibt die Stammlösung), 200 µl Insulin-Stammlösung (100 mg Insulin auf 10 ml HCl, 0,01 N) sowie den Wachstumsfaktoren EGF (20 ng/ml) und FGF 2 (20 ng/ml).

Das expandierte Gewebe wird homogenisiert, z.B. mit einer Eppendorffpipette, und die Zellzahl mit einem Hemozytometer bestimmt. Die Zellsuspension wird mit dem Expansionsmedium I oder II auf eine Zellzahl von ca. 300.000 Zellen/ml verdünnt. 8 ml dieser Zellsuspension (entsprechend ca. 2,5 Mill. Zellen) werden auf eine 25 cbm Flasche aufgesetzt und die Zellen mit einer Atmosphäre enthaltend 5 % CO₂ / 95 Luft bei 37° C für ca. eine Woche kultiviert.

Die Zellen werden ein bis zweimal pro Woche in frisches Expansionsmedium I oder II umgesetzt, wozu die Zellen in der Flasche abgelöst und in Plastikröhrchen überführt und anschließend zentrifugiert werden, der Überstand wird abgesaugt und 2 ml an der frischen Expansionslösung I oder II hinzugefügt und anschließend homogenisiert. Die homogenisierte Lösung wird in mehrere Proben, z.B. 3 bis 6 Proben, aufgeteilt, in neue Flaschen überführt und mit 8 ml der oben genannten Expansionslösung versetzt.

Die wie oben beschriebenen expandierten Progenitorzellen können zur Lagerung bei -80° tiefgefroren werden. Hierzu werden die Zellen aus der Kulturflasche mit der oben beschriebenen Trypsinlösung abgelöst und die Zellsuspension mit 1000xg für zehn Minuten zentrifugiert. Das nach Abgießen des Überstandes erhaltene Pellet wird in der Expansionslösung I oder II aufgenommen und die Zellsuspension mit der entsprechenden Expansionslösung bis auf eine Zellzahl von 2 bis 4 x 10⁶ je ml verdünnt. Die derart erhaltene Zellsuspension wird auf Eis zwischengelagert und anschließend 0,5 ml der Zellsuspension in ein bei -80° C vorgekühltes Einfrierröhrchen unter Zugabe von 0,5 ml eines Einfriermediums (bestehend aus 50% fetalem Kälberserum, 20% Dimetylsulfoxid und 30% der Expansionslösung I oder II) gefüllt. Die verschlossenen Röhrchen werden in einer Styroporbox bei -80° C eingefroren.

Zur Kontrolle werden die Proben am nächsten Tag oder später aufgetaut und bei ausreichender Zellaktivität die übrigen bei -80° C eingefrorenen Proben in einfrierfeste Folie eingeschweißt in flüssigen Stickstoff überführt.

Zur Kontrolle der eingefrorenen Zellproben werden die aufgetauten Zellen vermehrt, differenziert und mit den oben angegebenen, vom Zelltyp abhängigen Methoden charakterisiert. Die eingefrorenen Progenitorzellen, die ausreichende Zellcharakteristika aufweisen werden gelagert und als Transplantat verwendet.

Die derart präparierten Progenitorzellen können mit unbegrenzter Dauer in flüssigem Stickstoff gelagert werden.

Zum anschließenden Auftauen der Zellproben werden die Einfrierröhrchen direkt dem flüssigen Stickstoff entnommen und in einem Wasserbad bei 37° C vollständig aufgetaut. Anschließend wird das Röhrchen mit 70%-igem Ethanol desinfiziert. Die Zellsuspension in 15 ml-Plastikröhrchen überführt und mit 9 ml des Expansionsmediums I oder II langsam und unter Schütteln versetzt. Die entstandene Suspension wird bei 1000xg für zehn Minuten zentrifugiert, der Überstand abgegossen und das Zell-Pellet in 6 ml Expansionsmedium I oder II aufgenommen. Die Probe wird anschließend in eine 50 ml Kulturflasche überführt und in einer Atmosphäre mit 5 % CO₂ / 95 % Luft bei 37° C für ca. eine Woche inkubiert. Anschließend erfolgt die weitere Expansion der Zellen wie oben beschrieben.

Eine Differenzierung der Progenitorzellen kann mit frisch expandierten oder mit aufgetauten Proben erfolgen.

Die Zellen werden hierzu aus der Kulturflasche gespült, zentrifugiert und in einem geeigneten Aufnahmemedium aufgenommen, so daß eine Zellzahl von ca. 20.000 bis 100.000 Zellen/ml resultiert.

Nachfolgend werden beispielhaft die Zusammensetzungen dreier geeigneter Aufnahmemedien angegeben.
Das Aufnahmemedium I besteht aus 45,1 % F-12 Medium, 45,1 % DMEM, 1 ml Transferrin-Stammlösung, 1 ml Putrescin-Stammlösung (ergibt 60 µM), 150 I. E./ml Penicillin/150 µg/ml Streptomycin, 200 µl Na-Selenit-Stammlösung, 200 µl Progesterone-Stammlösung und 200 µl Insulin-Stammlösung.

Die Zusammensetzungen der genannten Stammlösungen entsprechen den Stammlösungen der oben beschriebenen Herstellung des Expansionsmediums II.

Das Aufnahmemedium II enthält 44,5 % F-12 Medium, 44,5 % DMEM, 150 I. E./ml Penicillin/150 µg/ml Streptomycin sowie 10% fetales Kälberserum (nicht Hitze-inaktiviert).

Das Aufnahmemedium III enthält 98 % eines Neurobasal Mediums (Firma Gibco), 1 % N-2 Supplement (Firma Gibco) sowie 150 I. E./ml Penicillin/150 µg/ml Streptomycin.

Den das Zellmaterial enthaltenden Aufnahmemedien werden zur Differenzierng jeweils IL-1 (100 pg/ml), IL-11 (1 ng/ml), GDNF (1 µg/ml) und LIF (1 ng/ml) zusammen oder diese Stoffe in den Angegebenen Konzentrationen jeweils einzeln zugesetzt.

Die Proben werden für 7 bis 21 Tage in einer Atmosphäre mit einem Sauerstoffgehalt von 2% inkubiert.

Anschließend werden die erhaltenen Zellen durch Subklonierung in einer Atmosphäre mit 5% Sauerstoff selektioniert, worauf die oben beschriebenen Schritte der Expansion und Differenzierung wiederholt werden.

Die entstandenen Zellsuspensionen werden für Transplantationen in einer phosphat-gepufferten Salzlösung aufgenommen.

Es wurden jeweils Präparate aus dopaminergen Neuronen erhalten, die praktisch frei von glialen Zellen sind. Die Zellen können dann transplantiert werden.

Es können auch jeweils Suspensionen determinierter Progenitorzellen, die ohne weitere Behandlung nach Implantation in das menschliche Gehirn oder bei Kontaktierung mit einem anderen Zellmedium oder Trägermaterial in einen bestimmten Typ partiell oder vollständig differenzieren, hergestellt werden. Gegebenenfalls kann sich den genannten Verfahrensschritten jeweils auch eine endgültige Differenzierung der Neuronen anschließen.

## Patentansprüche

1. Verfahren zur Herstellung von transplantationsfähigem neuronalem Zellmaterial, enthaltend die folgenden Verfahrensschritte:
- Gewinnung von humanen Progenitorzellen aus fetalem oder adultem neuronalem Zellmaterial;
- Expansion der gewonnenen humanen Progenitorzellen unter vermindertem Sauerstoffgehalt der Atmosphäre;
- partielle in vitro-Differenzierung durch Behandlung der Progenitorzellen mit LIF, GDNF und/oder einem oder mehreren der Interleukine IL 1-11 unter vermindertem Sauerstoffgehalt der Atmosphäre;
- Selektionierung durch Subklonierung unter vermindertem Sauerstoffgehalt der Atmosphäre; und
- Expansion des Zellmaterials.

2. Verfahren nach Anspruch 1, **dadurch gekennzeichnet, daß** die Subklonierung durch eine oder mehrere in geeigneter zeitlicher Abfolge ausgeführte Verfahren aus der folgenden Gruppe erfolgt:
- Subklonierung durch Endverdünnung;
- Subklonierung durch Mikromanipulation markierter vitaler Zellen;
- Subklonierung durch fluoreszenzaktivierte Zellsortierung markierter vitaler Zellen;
- Subklonierung durch magnetische Aufkonzentrierung von magnetisch markierten Zellen.

3. Verfahren nach Anspruch 1 oder 2, **dadurch gekennzeichnet, daß** der Sauerstoffgehalt der Atmosphäre < 10 Vol.-%, vorzugsweise < 5 Vol.-%, beträgt.

4. Verfahren nach einem der Ansprüche 1 bis 3, **dadurch gekennzeichnet, dass** zur partiellen Differenzierung der Progenitorzellen monoklonale Progenitorzellinien eingesetzt werden.

## Claims

1. A method for preparing transplantable neuronal cell material, containing the following method steps:
- deriving human progenitor cells from fetal or adult neuronal cell material;
- expanding the derived human progenitor cells under reduced oxygen content of the atmosphere;
- partial in vitro differentiation by treating the progenitor cells with LIF, GDNF and/or one or more of interleukines IL 1-11 under reduced oxygen content of the atmosphere;
- selection by subcloning under reduced oxygen content of the atmosphere; and
- expanding the cell material.

2. The method according to claim 1, **characterized in that** subcloning is effected by carrying out in a suitable sequence one or more procedures from the following group:
- subcloning using final dilution;
- subcloning using micromanipulation of labeled viable cells;
- subcloning using fluorescence-activated cell sorting of labeled viable cells;
- subcloning using magnetic concentration of magnetically labeled cells.

3. The method according to claim 1 or 2, **characterized in that** the oxygen content of the atmosphere is < 10 vol.-%, preferably < 5 vol.-%.

4. The method according to one of claims 1 to 3, **characterized in that** monoclonal progenitor cell lines are used for partial differentiation of the progenitor cells.

## Revendications

1. Procédé de production de matériau cellulaire neuronal pouvant être greffé, comprenant les étapes suivantes :
- recueil de cellules progénitrices humaines à partir d'un matériau cellulaire neuronal foetal ou adulte ;
- expansion des cellules progénitrices humaines obtenues sous une atmosphère à teneur réduite en oxygène ;
- différentiation partielle *in vitro* par traitement des cellules progénitrices avec LIF, GDNF et/ou une ou plusieurs des interleukines IL 1 à 11 sous atmosphère à teneur réduite en oxygène ;
- sélection par sous-clonage sous une atmosphère à teneur réduite en oxygène ; et
- expansion du matériau cellulaire.

2. Procédé selon la revendication 1, **caractérisé en ce que** le sous-clonage s'effectue par un ou plusieurs procédés réalisés dans un ordre temporel approprié compris dans le groupe suivant :
- sous-clonage par dilution terminale;
- sous-clonage par micromanipulation de cellules vitales marquées ;
- sous-clonage par tri cellulaire activé par fluorescence des cellules vitales marquées ;
- sous-clonage par concentration magnétique de cellules marquées magnétiquement.

3. Procédé selon la revendication 1 ou 2, **caractérisé en ce que** la teneur en oxygène de l'atmosphère est < à 10 % en volume, de préférence < 5 % en volume.

4. Procédé selon l'une quelconque des revendications 1 à 3, **caractérisé en ce que** pour la différenciation partielle des cellules progénitrices, on utilise des lignées de cellules progénitrices monoclonales.
